# EUROPÄISCHE PATENTSCHRIFT

(11) **EP 0 583 743 B1**
(45) Veröffentlichungstag und Bekanntmachung des Hinweises auf die Patenterteilung: **15.01.1997**
(21) Anmeldenummer: 93112942.3
(22) Anmeldetag: 12.08.1993
(51) Int. Cl.: C07C 69/82, C07C 67/03, C07C 67/02

(54) **Verfahren zur Herstellung von monomeren Terephthalsäurediestern und Diolen aus Polyestern**
Process for the production of monomeric terephthalic acid diesters and diols from polyesters
Procédé de préparation de diesters d'acide téréphthalique monomères et de diols à partir de polyesters

(30) Priorität: 18.08.1992 DE 4227299
(43) Veröffentlichungstag der Anmeldung: 23.02.1994
(73) Patentinhaber: HOECHST AKTIENGESELLSCHAFT, 65929 Frankfurt am Main (DE)
(72) Erfinder: Hertenstein, Ulrich, Dr., D-86456 Gablingen (DE); Neugebauer, Rudolf, D-86405 Meitingen (DE)

(56) Entgegenhaltungen:
- DE-A- 1 003 714
- US-A- 3 257 335
- US-A- 5 051 528

## Beschreibung

Die vorliegende Erfindung betrifft ein neues Verfahren zur Herstellung von Terephthalsäurediestern und Diolen aus Polyesterabfällen.

Neben Polyethylenterephthalat (im folgenden PET genannt) sind auch andere Polyester auf dem Markt erhältlich. Unter dem Begriff "Polyester" ist im folgenden ein Polyester zu verstehen, dessen Säurekomponente zumindest zum Teil Terephthalsäureeinheiten enthält.

Von der heute weltweit erzeugten Menge an PET von etwa 16 Millionen Tonnen pro Jahr wird nur ein verschwindend kleiner Anteil einem chemischen Recycling zugeführt. Andererseits bindet der erzeugte Polyester hohe Kapazitäten der Müllbeseitigung. Der Wiedereinsatz von Polyester nach Gebrauch als Polymer ist nur bedingt möglich, das heißt es können nur relativ kleine Mengen für niederwertige Anwendungszwecke untergebracht werden ("Down-Cycling").

Somit besteht Bedarf an geeigneten Verfahren, um Polyester chemisch so abzubauen, daß die daraus gewonnenen Monomeren, nach Reinigung, gleichwertig mit den üblichen Rohstoffen eingesetzt werden können ("Up-Cycling").

Prinzipiell kann Polyester zu Terephthalsäure oder zu einem Diester der Terephthalsäure, wie z.B. zu Dimethylterephthalat (im folgenden DMT genannt) umgesetzt werden. Terepthalsäure kann nur durch Umkristallisation und/oder Adsorption gereinigt werden, wobei die Filtration der gesamten Lösung für größere Durchsätze technisch äußerst aufwendig ist. Schwerlösliche Verunreinigungen können deshalb von Terephthalsäure nicht in erforderlichem Maße abgetrennt werden, was die Verwendbarkeit der erzeugten Terephthalsäure einschränkt. DMT kann sowohl umkristallisiert als auch destilliert werden. Es läßt sich deshalb, auch wenn es aus PET erzeugt wurde, auf die beim Polykondensationsprozeß hochwertiger Typen notwendige Reinheit bringen.

Da das Recycling von Polyester über DMT wegen der günstigeren Reinigungsmöglichkeiten dem über Terephthalsäure überlegen ist, wurde nach einem wirtschaftlichen Verfahren gesucht, das kontinuierlich arbeitet und große Mengen an Polyester unter möglichst milden Bedingungen umsetzen kann.

Es ist bekannt, daß Polyester mit Methanol bei Temperaturen zwischen 100 und 300 °C und Drücken bis zu 150 atm zu DMT abgebaut werden kann (US-A-3,776,945). Dieses Verfahren hat den Nachteil, daß sehr lange Reaktionszeiten neben hohen Drücken in Kauf genommen werden müssen. Mildern kann man diesen Mangel sowohl durch den Einsatz von Dampf (US-A-3,321,510), als auch von Umesterungskatalysatoren (US-A-3,037,050).

In der DE-A-1,003,714 wurde beschrieben, daß der Abbau von Polyester zu DMT mit Methanol und Umesterungskatalysatoren durch die Gegenwart von DMT stark beschleunigt wird. Analog dazu beschreibt die US-A-5,051,528, daß die Umsetzung von PET zu DMT in Gegenwart von Umesterungskatalysatoren mit Methanol dadurch beschleunigt wird, daß man das PET zu Oligomeren zudosiert und gleichzeitig mit überkritischem Methanoldampf begast. Die Reaktion wird so durchgeführt, daß DMT, Glykol und überschüssiges Methanol abdestilliert werden. Nichtflüchtige Bestandteile im PET reichern sich daher im Reaktor an. So muß bei kontinuierlicher Reaktionsführung ein großer Ausschleusestrom in Kauf genommen werden.

Die vorbekannten Verfahren zur Rückgewinnung der Säure- und der Alkoholkomponente aus Polyestern sind also durch die Anwendung aufwendiger Verfahrensmaßnahmen gekennzeichnet. So muß nach US-A-5,051,528 festes PET in einen, mit überkritischem Methanol begasten, unter Druck stehenden Reaktor gebracht werden, will man die Reaktion kontinuierlich führen. Nach der DE-A-1,003,714 ist eine kontinuierliche Fahrweise nicht möglich.

Beiden Verfahren gemeinsam ist, daß der PET-Abbau mit Methanol in Anwesenheit von DMT oder Oligomeren erfolgt.

Ausgehend von diesen vorbekannten Verfahren lag der vorliegenden Erfindung die Aufgabe zugrunde, ein Verfahren zu entwickeln, das in der Lage ist, große Mengen an Polyester rasch, unter möglichst milden Bedingungen zu Terephthalsäurediester und Diolen, insbesondere zu DMT und Ethylenglykol, umzusetzen, unter Vermeidung der oben beschriebenen verfahrenstechnischen Schwierigkeiten und das besonders vorteilhaft kontinuierlich betrieben werden kann.

Überraschenderweise wurde nun gefunden, daß sich Polyester mit Estern einwertiger Alkohole in Gegenwart von Umesterungskatalysatoren depolymerisieren lassen, wobei die Depolymerisation vorzugsweise drucklos erfolgt. Man erhält z.B. aus DMT und PET bei einer Stöchiometrie von 1 zu 1 in Bezug auf den Aromaten Dimethylethylenditerephthalat (im folgenden DMEDT genannt). Wird DMT im Unterschuß zu PET eingesetzt, so erhält man Oligomere mit Methylestergruppen als Endgruppe. Oligomere und DMEDT lassen sich in einem Umesterungsgleichgewicht mit Methanol zu DMT umestern. Es wurde gefunden, daß die Depolymerisationsstufe in Gegenwart eines Umesterungskatalysators stark beschleunigt abläuft. Führt man die Reaktion zwischen Polymer und Ester in Gegenwart von Umesterungskatalysatoren durch, so kann die Reaktion bereits drucklos erfolgen und, je nach vorgegebener Reaktionstemperatur, innerhalb von Minuten beendet sein. Durch die Trennung der beiden Reaktionen, Polymerabbau und Umesterung, läßt sich ein kontinuierliches Verfahren mit einfacher Technologie entwickeln.

Die Erfindung betrifft daher ein Zweistufenverfahren zur Herstellung von monomeren Terephthalsäurediestern und Diolen durch Depolymerisation eines Polyesters mit Estern einwertiger Alkohole in Gegenwart von Umesterungskatalysatoren und anschließender Umesterung des Depolymerisats mit einem einwertigen Alkohol.

Zur Durchführung des erfindungsgemäßen Verfahrens trägt man PET-Granulat in Gegenwart von Umesterungskatalysatoren vorzugsweise drucklos z.B. in eine DMT-Schmelze ein; anhaftendes Wasser wird größtenteils verdampft. Dabei können die Umesterungskatalysatoren aus dem PET-Granulat stammen und/oder zugesetzt werden. Das Granulat löst sich unter Abbau und man erhält eine pumpfähige Schmelze. Diese wird einem Umesterungsreaktor zugeführt, der von unten mit Methanol begast wird. Methanolüberschuß und erzeugtes Ethylenglykol werden über Kopf des Reaktors geführt, während DMT und Verunreinigungen aus dem PET über Sumpf abgezogen werden. Das Sumpfprodukt wird zur weiteren Reinigung destilliert. Das Kopfprodukt wird destillativ aufgetrennt.

Ein wesentlicher Vorteil dieses Verfahrens ist die Tatsache, daß das bei der Depolymerisation eingebrachte Wasser größtenteils verdampft wird. Partiell tritt Hydrolyse ein, unter Freisetzung von Methanol. Die erzeugten Säuren werden auf der Umesterungsstufe unter Wasserbildung wieder verestert. Beim Einstufenprozeß nach US-A-5,051,528 fällt dagegen alles mit dem PET-Granulat eingebrachte Wasser mit dem Methanol-Glykolgemisch an und muß destillativ abgetrennt werden.

Als Ausgangsmaterial lassen sich alle terephthalsäurehaltigen Polyester einsetzen. Dabei handelt es sich also um Homo- oder Copolyester, die Terephthalsäure als Säurekomponente enthalten. Bevorzugte Beispiele sind Polybutylenterephtalat und insbesondere Polyethylenterephtalat. Beispiele für Copolyester sind Copolyethylenterephtalate, die neben Terephthalsäure und Ethylenglykol aliphatische Dicarbonsäuren, wie Adipinsäure oder Sebacinsäure als Säurekomponente enthalten, und/oder die aliphatische Diole, wie Diethylenglykol oder Butylenglykol als Alkoholkomponente enthalten.

Als Ester im Depolymerisationsschritt wird vorzugsweise ein Alkylester, insbesondere ein Dialkylester einer aromatischen Dicarbonsäure, eingesetzt.

Beispiele für Alkylester sind Alkylester von aliphatischen, cycloaliphatischen, araliphatischen oder insbesondere aromatischen Mono- oder Dicarbonsäuren. Bevorzugt werden die Ester mit Alkylgruppen mit ein bis sechs Kohlenstoffatomen, insbesondere die Ethyl- und ganz besonders die Methylester.

Als Säurekomponente für die Alkylester eignen sich beispielsweise Ameisensäure, Essigsäure, Propionsäure, Buttersäure, Adipinsäure, Sebacinsäure oder Cyclohexancarbonsäure. Insbesondere eignen sich als Säurekomponente für die Alkylester aromatische Dicarbonsäuren, wie Isophthalsäure, Phthalsäure und insbesondere Terephthalsäure.

Beispiele für besonders bevorzugte Ester sind Dimethylisophthalat und ganz besonders Dimethylterephthalat.

Der Ester wird im allgemeinen in einer Menge von 0,5 bis 10 Mol, bezogen auf ein Mol Polyester, eingesetzt. Bevorzugt verwendet man 1 bis 5 Mol.

Es lassen sich auch Mischungen von Estern einsetzen.

Als Umesterungskatalysatoren für die Depolymerisation lassen sich alle für diesen Zweck geeigneten Verbindungen oder Gemische solcher Verbindungen einsetzen.

Beispiele für bevorzugte Umesterungskatalysatoren sind Manganacetat, Zinkoxid, Zinkacetat, Zinkchlorid oder Magnesiumoxid oder Gemische solcher Verbindungen, sowie andere saure Umesterungskatalysatoren.

Die Katalysatoren werden zweckmäßigerweise in Mengen von 10 bis 500 ppm, bezogen auf den Polyester, eingesetzt.

Es wurde gefunden, daß die Depolymerisation in nennenswertem Umfang ab Schmelzetemperatur des eingesetzten Esters abläuft. Im Falle von DMT wählt man daher eine Depolymerisationstemperatur von mindestens 140 °C. Die Reaktionsgeschwindigkeit nimmt mit der Temperatur zu. Die Löslichkeit von Wasser in DMT mit der Temperatur ab.

Im Falle von DMT wählt man als Temperaturbereich für die Depolymerisation vorzugsweise 140 bis 300 °C, insbesondere 140 bis 250 °C. Die Reaktion ist auch bei druckloser Durchführung innerhalb weniger Minuten abgeschlossen. Mit eingebrachtes Wasser wird größtenteils abdestilliert.

Die Depolymerisation kann in Lösung oder vorzugsweise in der Schmelze erfolgen; sie kann vorzugsweise drucklos oder unter Druck, beispielsweise bei einem Druck von 1 bis 30 bar, vorzugsweise 1 bis 5 bar, durchgeführt werden.

Als Lösungsmittel verwendet man beispielsweise DMSO, DMF, N-Methylpyrrolidon, Hexamethylenphosphorsäuretriamid oder andere Lösemittel, in denen der Polyester wenigstens teilweise löslich ist.

Das Fortschreiten der Reaktion läßt sich zweckmäßigerweise durch die Überwachung der Schmelzeviskosität oder Lösungsviskosität im Reaktionsgemisch verfolgen. Das Gleichgewicht in der Schmelze ist üblicherweise erreicht, sobald der Viskositätswert in der Nähe des Wertes für den eingesetzten Ester liegt, bei der Wahl einer Stöchiometrie, so daß das PET vollständig abgebaut werden kann, also bei einer Stöchiometrie von DMT : PET > 1 : 1. Selbstverständlich kann der Abbau in der ersten Stufe, insbesondere bei kontinuierlichem Betrieb, auch bei höheren Viskositätswerten gehalten werden.

Zur Umesterung lassen sich im Prinzip im erfindungsgemäßen Verfahren praktisch alle einwertigen Alkohole oder Gemische einwertiger Alkohole einsetzen. Dabei handelt es sich also um einwertige cycloaliphatische, araliphatische, aromatische oder insbesondere aliphatische Alkohole.

Beispiele für solche Alkohole sind Cyclohexanol, Methylolcyclohexan, Phenol, Methanol, Ethanol, Propanol, Butanol, Pentanol und Hexanol.

Bevorzugt setzt man in der Umesterungsstufe einen Alkylalkohol mit ein bis sechs Kohlenstoffatomen, insbesondere Methanol, ein.

Der einwertige Alkohol wird im allgemeinen in einer Menge von 2 bis 20 Mol, bezogen auf ein Mol Polyester, eingesetzt. Bevorzugt verwendet man 5 bis 10 Mol. Der einwertige Alkohol wird nach Trennung vom erzeugten Diol vorzugsweise in der Veresterungsstufe im Kreis gefahren.

Die Umesterungsstufe kann bei Temperaturen zwischen 140 und 300 °C und drucklos oder unter Überdruck, beispielsweise zwischen 1 bis 20 bar, betrieben werden.

Ganz besonders bevorzugt werden die beiden Verfahrensschritte kontinuierlich durchgeführt.

In einer besonders bevorzugten Variante des erfindungsgemäßen Verfahrens wird ein Teil des gewonnenen Diesters der Terephthalsäure in die Depolymerisationsstufe zurückgeführt.

Die folgenden Beispiele beschreiben das Verfahren.

### Beispiel 1:

In einen Durchflußreaktor werden gleichzeitig folgende Ströme zudosiert: 3 Mole flüssiges DMT, ein Mol PET-Granulat und 300 ppm Manganacetat. Die Reaktionstemperatur beträgt 200 °C die mittlere Verweilzeit 40 Minuten.

Das erzeugte Produktgemisch aus DMEDT in DMT wird kontinuierlich einer Umesterungskolonne aufgegeben, in die von unten Methanoldampf eingespeist wird. Über Kopf wird kontinuierlich ein Methanol-Glykolgemisch abgezogen, das teilweise als Rücklauf dient und teilweise über eine weitere Kolonne aufgetrennt wird. Glykol wird abgetrennt; Methanol wird der Umesterungskolonne wieder zugeführt. Im Sumpf der Umesterungskolonne wird kontiniuierlich DMT entnommen, von dem ein Teil in den Durchflußreaktor zurückgeführt wird und der andere Teil einer Rektifikationskolonne aufgegeben wird.

### Beispiel 2:

In einen Durchflußreaktor werden kontinuierlich folgende Ströme zudosiert: 1,5 Mole DMT, ein Mol PET-Granulat und 400 ppm festes Manganacetat. Die Reaktionstemperatur beträgt 180 °C, der Reaktionsdruck 5 bar. Die mittlere Verweilzeit beträgt 30 Minuten. Das erhaltene Produktgemisch wird kontinuierlich nach Beispiel 1 weiterverarbeitet.

## Patentansprüche

1. Verfahren zur Herstellung von Terephthalsäurediestern und Diolen durch Depolymerisation eines Polyesters in Gegenwart von Umesterungskatalysatoren für besagten Polyester, dadurch gekennzeichnet, daß die Depolymerisation in Gegenwart eines Esters erfolgt und daß anschließend das Gemisch durch Umesterung mit einem einwertigen Alkohol zu Terephthalsäurediester und Diol umgesetzt wird.

2. Verfahren nach Anspruch 1, dadurch gekennzeichnet, daß als Polyester Polyethylenterephthalat eingesetzt wird.

3. Verfahren nach einem der Ansprüche 1 oder 2, dadurch gekennzeichnet, daß als Ester im Depolymerisationsschritt ein Alkylester, insbesondere ein Dialkylester einer aromatischen Dicarbonsäure, eingesetzt wird.

4. Verfahren nach Anspruch 3, dadurch gekennzeichnet, daß es sich bei dem Ester um Dimethylterephthalat handelt.

5. Verfahren nach Anspruch 1, dadurch gekennzeichnet, daß man als Umesterungskatalysator Manganacetat, Zinkoxid, Zinkacetat, Zinkchlorid oder Magnesiumoxid oder ein Gemisch solcher Verbindungen einsetzt.

6. Verfahren nach Anspruch 2, dadurch gekennzeichnet, daß man die Depolymerisation bei Temperaturen zwischen 140 und 250 °C drucklos in Gegenwart der Umesterungskatalysatoren nach Anspruch 5 durchführt.

7. Verfahren nach Anspruch 1, dadurch gekennzeichnet, daß die Reaktionskontrolle durch Messen der Schmelzeviskosität erfolgt.

8. Verfahren nach Anspruch 1, dadurch gekennzeichnet, daß es sich bei dem einwertigen Alkohol in der Umesterungsstufe um einen Alkylalkohol mit ein bis sechs Kohlenstoffatomen, insbesondere um Methanol, handelt.

9. Verfahren nach Anspruch 1, dadurch gekennzeichnet, daß man die Umesterung bei Temperaturen zwischen 140 und 300 °C und bei Drücken zwischen 1 und 20 bar durchführt.

10. Verfahren nach Anspruch 1, dadurch gekennzeichnet, daß man aus der Umesterungsstufe überschüssigen einwertigen Alkohol zusammen mit dem aus dem Polyester stammenden Diol abdestilliert, und der im Rückstand verbleibende Terephthalsäurediester anschließend destillativ gereinigt wird.

11. Verfahren nach Anspruch 1, dadurch gekennzeichnet, daß man die Depolymerisationsstufe und die Umesterungsstufe kontinuierlich betreibt.

12. Verfahren nach Anspruch 1, dadurch gekennzeichnet, daß ein Teil des gewonnenen Diesters der Terephthalsäure in die Depolymerisationsstufe zurückgeführt wird.

## Claims

1. A process for the preparation of terephthalic diesters and diols by depolymerization of a polyester in the presence of transesterification catalysts for said polyester, which comprises carrying out the depolymerization in the presence of an ester and subsequently converting the mixture into terephthalic diester and diol by transesterification by means of a monohydric alcohol.

2. The process as claimed in claim 1, wherein the polyester employed is polyethylene terephthalate.

3. The process as claimed in claim 1 or 2, wherein the ester employed in the depolymerization step is an alkyl ester, in particular a dialkyl ester of an aromatic dicarboxylic acid.

4. The process as claimed in claim 3, wherein the ester is dimethyl terephthalate.

5. The process as claimed in claim 1, wherein the transesterification catalyst employed is manganese acetate, zinc oxide, zinc acetate, zinc chloride or magnesium oxide, or a mixture of these compounds.

6. The process as claimed in claim 2, wherein the depolymerization is carried out at temperatures between 140 and 250°C at atmospheric pressure in the presence of the transesterification catalysts as claimed in claim 5.

7. The process as claimed in claim 1, wherein the reaction monitoring is carried out by measuring the melt viscosity.

8. The process as claimed in claim 1, wherein the monohydric alcohol in the transesterification step is an alkyl alcohol having one to six carbon atoms, in particular methanol.

9. The process as claimed in claim 1, wherein the transesterification is carried out at temperatures between 140 and 300°C and at pressures between 1 and 20 bar.

10. The process as claimed in claim 1, wherein excess monohydric alcohol is removed from the transesterification step by distillation together with the diol originating from the polyester, and the terephthalic diester remaining in the residue is subsequently purified by distillation.

11. The process as claimed in claim 1, wherein the depolymerization step and the transesterification step are carried out continuously.

12. The process as claimed in claim 1, wherein some of the terephthalic diester obtained is fed back into the depolymerization step.

## Revendications

1. Procédé pour préparer des diesters de l'acide téréphtalique et des diols par dépolymérisation d'un polyester en présence de catalyseurs de transestérification desdits polyesters, caractérisé en ce que la dépolymérisation est réalisée en présence d'un ester, puis que le mélange est, par transestérification avec un monoalcool, converti en un diester de l'acide téréphtalique et en un diol.

2. Procédé selon la revendication 1, caractérisé en ce qu'on utilise comme polyester le poly(téréphtalate d'éthylène).

3. Procédé selon l'une des revendications 1 ou 2, caractérisé en ce qu'on utilise comme ester dans l'étape de dépolymérisation un ester alkylique, notamment un ester dialkylique d'un acide dicarboxylique aromatique.

4. Procédé selon la revendication 3, caractérisé en ce que, pour ce qui est de l'ester, il s'agit du téréphtalate de diméthyle.

5. Procédé selon la revendication 1, caractérisé en ce qu'on utilise comme catalyseur de transestérification l'acétate de manganèse, l'oxyde de zinc, l'acétate de zinc, le chlorure de zinc ou l'oxyde de magnésium ou un mélange de ces composés.

6. Procédé selon la revendication 2, caractérisé en ce qu'on met en oeuvre la dépolymérisation à des températures comprises entre 140 et 250°C, sans pression, en présence des catalyseurs de transestérification selon la revendication 5.

7. Procédé selon la revendication 1, caractérisé en ce que la réaction est suivie par une mesure de la viscosité à l'état fondu.

8. Procédé selon la revendication 1, caractérisé en ce que, pour ce qui est du monoalcool de l'étape de transestérification, il s'agit d'un alcool alkylique ayant de 1 à 6 atomes de carbone, en particulier le méthanol.

9. Procédé selon la revendication 1, caractérisé en ce qu'on met en oeuvre la transestérification à des températures de 140 à 300°C et sous des pressions de 1 à 20 bar.

10. Procédé selon la revendication 1, caractérisé en ce qu'on chasse par distillation le monoalcool en excès provenant de l'étape de transestérification, en même temps que le diol provenant du polyester, puis on purifie par distillation le diester de l'acide téréphtalique restant dans le résidu.

11. Procédé selon la revendication 1, caractérisé en ce que l'étape de dépolymérisation et l'étape de transestérification sont exploitées en continu.

12. Procédé selon la revendication 1, caractérisé en ce qu'une partie du diester obtenu de l'acide téréphtalique est renvoyée à l'étape de dépolymérisation.
